# FASCICULE DE BREVET EUROPEEN

(11) **EP 3 215 199 B1**
(45) Date de publication et mention de la délivrance du brevet: **12.09.2018**
(21) Numéro de dépôt: 15798524.3
(22) Date de dépôt: 30.10.2015
(51) Int. Cl.: A61M 1/06

(54) **ENSEMBLE D'EXPRESSION DE LAIT MATERNEL**
ANORDNUNG ZUM ABPUMPEN VON BRUSTMILCH
ASSEMBLY FOR EXPRESSING BREASTMILK

(30) Priorité: 05.11.2014 FR 1460682
(43) Date de publication de la demande: 13.09.2017
(73) Titulaire: LA DIFFUSION TECHNIQUE FRANCAISE, 42000 Saint Etienne (FR)
(72) Inventeur: CHANTREL, Gilles, F-42100 Saint-Etienne (FR); MASSARDIER, Jean-Philippe, F-42100 Saint-Etienne (FR)
(74) Mandataire: Cabinet Laurent & Charras
(86) Numéro de dépôt international: PCT/FR2015/052926
(87) Numéro de publication internationale: WO 2016/071609

(56) Documents cités:
- FR-A1- 2 985 188
- US-A- 4 892 517
- US-A- 5 843 029
- US-A1- 2005 228 342
- US-A1- 2007 060 873

## Description

L'invention se rattache au secteur technique d'un ensemble d'expression de lait maternel.

De manière parfaitement connue pour un Homme du Métier, cet ensemble comprend pour l'essentiel une téterelle et un contenant généralement sous forme d'un biberon. La téterelle est en communication avec le biberon par l'intermédiaire d'un clapet anti-retour. La téterelle comprend, toujours de manière connue, un embout en matière souple apte à être appliqué au niveau d'un mamelon. A l'opposé de l'embout, le corps tubulaire de la téterelle présente une embase circulaire faisant office de bouchon apte à être vissé sur une portée filetée externe formée à l'extérieur d'un col que présente l'ouverture du biberon.

Quelle que soit la forme de l'ensemble d'expression, le clapet anti-retour est fixé au niveau de la téterelle. Par exemple, ce clapet est fixé sur un bouchon cylindrique formé coaxialement dans le volume interne de la portée circulaire faisant office de manchon. Ce manchon est en communication avec le corps de la téterelle par l'intermédiaire d'un orifice et également en communication par l'intermédiaire d'un autre orifice à un embout de raccordement avec un appareil de tout type connu et approprié, dont la fonction est de tirer le lait.

Le clapet anti-retour est généralement réalisé dans un matériau élastomère et peut se présenter sous la forme d'une bague prolongée par deux lèvres en regard finissant une ouverture de passage du lait. Ce clapet est monté de manière amovible en vue de son nettoyage après utilisation. Il apparait donc qu'après nettoyage et avant une nouvelle utilisation, le clapet doit être mis en place au niveau de la téterelle comme indiqué. Il est évident qu'une telle manoeuvre génère des risques importants de contamination par l'utilisateur, et par conséquent une contamination du lait.

Pour tenter de remédier à ces inconvénients, il est proposé une solution comme il ressort de l'enseignement du brevet FR 2985188 dont le demandeur de la présente est également titulaire. Selon l'enseignement de ce brevet, le clapet anti-retour est accouplé, au niveau de l'embase de la téterelle, par l'intermédiaire d'un manchon de protection qui entoure totalement le clapet et fait office de moyen de préhension lors du montage dudit clapet sur la téterelle. Cette solution apporte une amélioration significative mais ne peut toutefois être considérée comme vraiment satisfaisante, car on n'évite pas totalement le risque de contamination. En effet, l'extérieur du manchon peut être contaminé par l'utilisateur au moment du montage sur la base de la téterelle. Or, si le biberon est incliné après avoir recueilli le lait, ce dernier peut venir en contact avec l'extérieur du manchon.

L'invention s'est fixée pour but de remédier à ces inconvénients d'une manière simple, sûre, efficace et rationnelle.

Le problème que se propose de résoudre l'invention est de permettre à l'utilisateur d'éviter tout contact avec le clapet anti-retour afin de supprimer tout risque de contamination.

Pour résoudre un tel problème, il a été conçu et mis au point, un ensemble d'expression de lait maternel comprenant, de manière connue, une téterelle vissée sur une portée filetée formée à l'extérieur d'un col que présente une ouverture d'un biberon, ladite téterelle étant en communication avec le biberon par l'intermédiaire d'un clapet anti-retour. Selon l'invention, le clapet anti-retour est rendu solidaire d'un organe de protection présentant des agencements pour être accouplé d'une manière amovible, au niveau de l'ouverture du biberon.

Il ressort donc de ces caractéristiques que, contrairement aux différentes solutions de l'état antérieur de la technique, le clapet anti-retour est monté non pas au niveau de la téterelle mais directement au niveau du contenant faisant office de biberon. Pour résoudre le problème posé de permettre à l'utilisateur de monter le clapet anti-retour au niveau du biberon sans toucher ledit clapet ou le manchon de protection entourant le clapet, les agencements de l'organe tubulaire de protection sont constitués par une portée circulaire apte à être emmanchée à force dans l'ouverture du col du biberon, ladite portée étant formée coaxialement à un manchon cylindrique entourant le clapet anti-retour. Avantageusement, 1a portée circulaire présente une collerette apte à prendre appui sur le rebord de l'ouverture après emmanchement de ladite portée dans l'ouverture du col du biberon.

Il résulte de ces caractéristiques que lors du nettoyage le clapet anti-retour de protection repose naturellement sur la collerette. Dans ces conditions, l'utilisateur peut tout simplement saisir manuellement le biberon et le renverser afin de l'emboiter directement sur la portée circulaire d'emmanchement sans aucun contact des doigts sur ledit organe tubulaire, notamment le manchon de protection du clapet. On note également que l'utilisateur peut saisir l'organe tubulaire par le bord circulaire externe de la collerette afin d'emmancher ledit corps sur l'ouverture du col du biberon. Le contact avec les doigts se retrouve à l'extérieur du biberon et ne peut, en aucun cas, être en contact avec le lait. Il apparait donc, quel que soit le mode opératoire pour l'emmanchement de l'organe tubulaire dans le biberon, que le risque de contamination est totalement évité.

Pour résoudre le problème posé de mise en place du clapet anti-retour, le manchon et la portée circulaire d'emmanchement délimitent un épaulement interne faisant office de siège pour la fixation dudit clapet anti-retour.

Selon une autre caractéristique, la périphérie externe de la portée d'emmanchement présente des zones ponctuelles en creux et/ou relief pour améliorer l'accouplement.

Selon une autre caractéristique, la collerette coopère en appui avec un point circulaire d'étanchéité que présente la base de la téterelle faisant office de bouchon pour être vissé sur la portée filetée externe du col du biberon.

Selon une forme de réalisation, le clapet anti-retour coopère, après accouplement de l'organe tubulaire de protection sur le biberon, avec une portée circulaire interne que présente le volume intérieur de la base de la téterelle faisant office de bouchon, ladite portée interne étant, d'une manière connue, en communication avec la téterelle par un orifice pour réduire le passage de circulation et de transfert du lait dans le biberon.

L'organe tubulaire de protection incluant le manchon, la portée circulaire d'emmanchement et la collette sont réalisés d'une manière monobloc dans une matière plastique alimentaire.

L'invention est exposée ci-après plus en détail à l'aide des figures des dessins annexés dans lesquels :

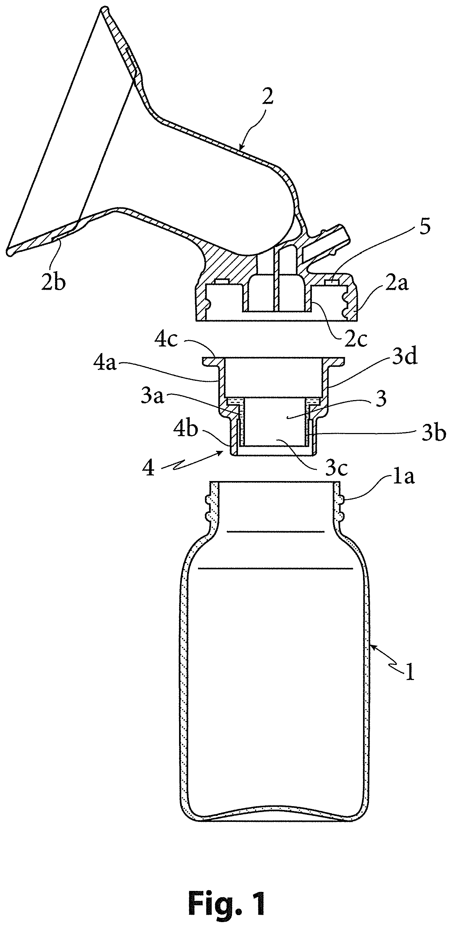

- la figure 1 est une vue éclatée avant montage selon une forme de réalisation de l'ensemble d'expression de lait maternel selon les caractéristiques de l'invention.
- la figure 2 est une vue correspondant à la figure 1 après accouplement des différents éléments constitutifs.
- la figure 3 est une vue en perspective de l'organe de protection recevant le clapet anti-retour.
- Les figures 4 et 5 sont des vues à caractère schématique montrant le mode opératoire pour l'emmanchement de l'organe tubulaire incluant le clapet anti-retour, sans risque de contamination dudit clapet.

On rappelle, pour une meilleure compréhension de la suite de la description, que l'ensemble d'expression de lait maternel comprend, de manière connue, un contenant (1) faisant office de biberon et un clapet anti-retour (3). Le corps de la téterelle (2) est en communication avec l'une de ses extrémités avec une embase circulaire (2a) et à son extrémité avec un embout souple (2b). L'embase circulaire (2a) fait office de bouchon et présente un alésage taraudé apte à être vissé sur une portée externe filetée du col (1a)) du biberon (1). Le volume interne de l'embase (2a) présente une portée interne circulaire (2c) formée concentriquement et coaxialement à ladite embase (2a) en étant en communication par un orifice avec le corps tubulaire de la téterelle et par un autre orifice à un embout pour être raccordé à un appareil du type tire-lait. L'embout souple (2b) est destiné à être appliqué au niveau du mamelon. Ces dispositions ne sont pas décrites en détail car parfaitement connues pour un Homme du Métier et susceptibles de faire l'objet d'autres moyens d'exécution.

Le clapet anti-retour (3) peut également être réalisé d'une manière connue en formant une bague (3a) prolongée par deux lèvres (3b) définissant une ouverture de passage du lait. Ce clapet (3) est réalisé dans un matériau élastomère avec capacité de déformation élastique.

Selon une caractéristique à la base de l'invention, le clapet anti-retour (3) est rendu solidaire d'un organe tubulaire de protection (4) présentant des agencements pour être accouplé de manière amovible au col (1a). Les agencements d'accouplement de l'organe de protection tubulaire (4) sont constitués par une portée circulaire (4a) apte à être emmanché à force dans l'ouverture du col (1a) du biberon. Cette portée (4a) est formée coaxialement à un manchon cylindrique (4b) entourant l'ensemble du clapet anti-retour (3). Avantageusement, la portée circulaire d'emmanchement (4a) présente une collerette (4c) faisant office de couronne apte à prendre appui sur le rebord de l'ouverture du col (1a), après emmanchement de ladite portée dans ledit col. Le diamètre externe de la collerette (4c) est égal ou légèrement inférieur au diamètre externe du col (1a).

La portée circulaire d'emmanchement (4a) est reliée au manchon de protection (4c) par un épaulement (4d) faisant office de siège pour la fixation du clapet (3) qui présente à cet égard une couronne d'appui (3d).

Il résulte de ces caractéristiques que l'utilisateur après avoir nettoyé le clapet (3) intégré au corps tubulaire (4), peut réinstaller ce dernier au niveau du col du biberon sans risque de contamination.

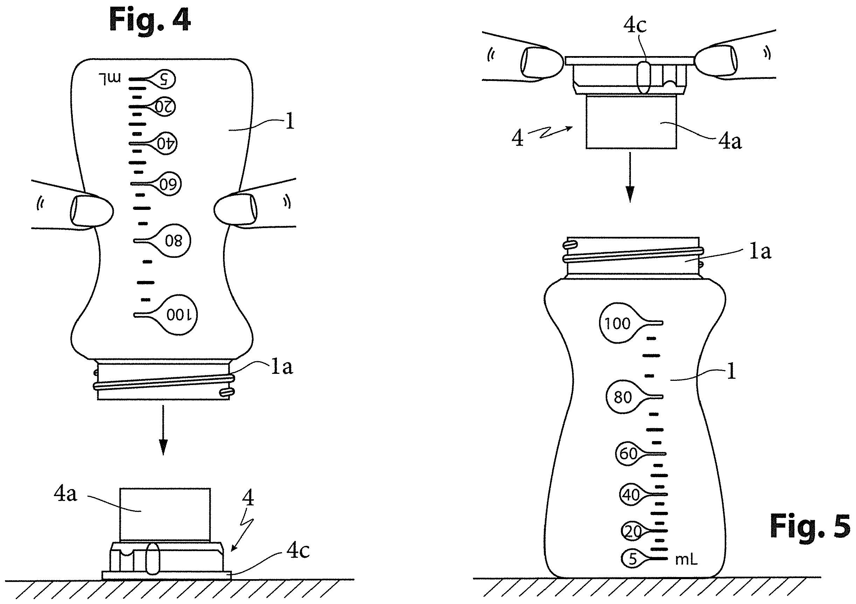

Par exemple, comme le montre la figure 4, l'organe tubulaire de protection (4) peut naturellement reposer sur un support quelconque au moyen de la collerette (4c). Il suffit dans ce cas de saisir manuellement le biberon (1), de le renverser et de l'emboiter directement sur la portée (4a) dudit corps (4). Avec ce mode opératoire, il n'y a donc aucun contact d'une quelconque partie de l'organe (4) et, à fortiori du clapet anti-retour (3), avec les doigts de l'utilisateur.

Selon le mode opératoire illustré à la figure 5, l'opérateur saisit l'organe tubulaire (4) par le bord circulaire externe de la collerette (4c) et emmanche ledit corps à l'intérieur du col (1a) du biberon. Le bord externe de la collerette (4c), qui a été en contact avec les doigts de l'utilisateur, se retrouve à l'extérieur du biberon et ne peut pas, par conséquent, être en contact avec le lait. Là encore, tout risque de contamination est évité.

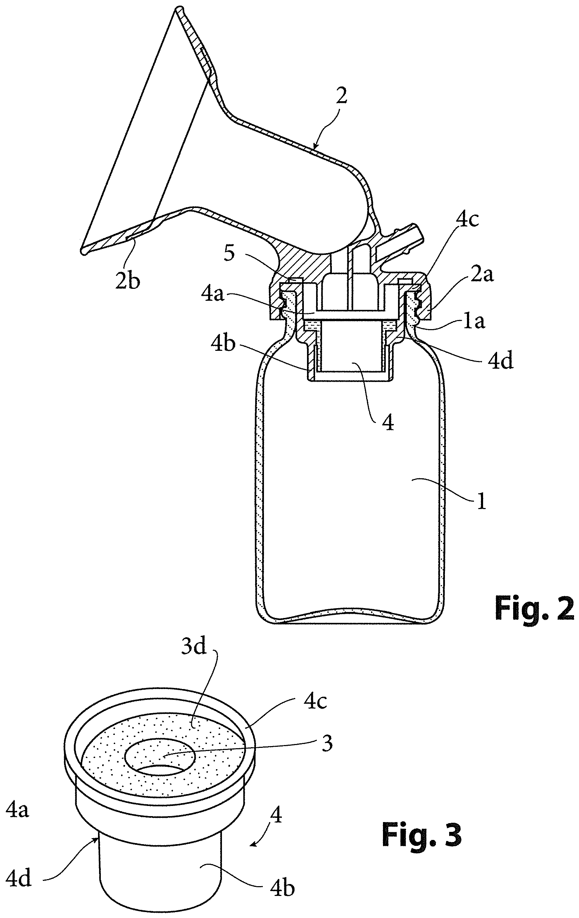

A noter que la périphérie externe de la portée d'emmanchement (4a) peut présenter des zones ponctuelles en creux et en relief pour améliorer l'accouplement. On voit également, figure 2, que la collerette (4c) coopère en appui avec un joint circulaire (5) monté dans le fonds de l'embase (2a) de la téterelle (2).

Selon une forme de réalisation appropriée, le clapet anti-retour (3) coopère après accouplement de l'organe tubulaire de protection (4) sur le biberon dans les conditions indiquées, avec la portée circulaire interne (2c) de l'embase (2a) de la téterelle (2).

L'organe tubulaire (4) incluant le manchon de protection (4b), la portée d'emmanchement (4a), et la collerette (4c), est réalisé de manière monobloc dans une matière plastique alimentaire de tout type connu et approprié.

Les avantages ressortent bien de la description, en particulier on souligne et on rappelle que les agencements de montage du clapet anti-retour au niveau du biberon et non plus au niveau de la téterelle, suppriment tout risque de contamination en évitant tout contact des doigts de l'utilisateur avec une partie quelconque susceptible d'être en contact avec le lait maternel.

## Revendications

1. Ensemble d'expression de lait maternel comprenant une téterelle (2) vissée sur une portée filetée formée à l'extérieur d'un col (1a) que présente une ouverture d'un biberon (1), ladite téterelle (2) étant en communication avec le biberon (1) par l'intermédiaire d'un clapet anti-retour (3), ***caractérisé* en ce que** le clapet anti-retour (3) est rendu solidaire d'un organe de protection (4) présentant une portée circulaire (4a) apte à être emmanchée à force dans l'ouverture du col (1a) du biberon, ladite portée (4a) étant formée coaxialement à un manchon cylindrique (4b) entourant le clapet anti-retour (3).

2. Ensemble d'expression de lait maternel selon la revendication 1, ***caractérisé* en ce que** la portée circulaire (4a) présente une collerette (4c) apte à prendre appui sur le rebord de l'ouverture du col du biberon (1), après emmanchement de ladite portée dans ladite ouverture.

3. Ensemble d'expression de lait maternel selon l'une quelconque des revendications 1 à 2, ***caractérisé* en ce que** le manchon (4b) et la portée circulaire d'emmanchement (4a), délimitent un épaulement interne faisant office de siège pour la fixation du clapet anti-retour (3).

4. Ensemble d'expression de lait maternel selon l'une quelconque des revendications 1 à 3, ***caractérisé* en ce que** la périphérie externe de la portée d'emmanchement (1a), présente des zones ponctuelles en creux et/ou relief pour améliorer l'accouplement.

5. Ensemble d'expression de lait maternel selon la revendication 2, ***caractérisé* en ce que** la collerette (4c) coopère en appui avec un joint circulaire d'étanchéité (5) que présente une embase (2a) de la téterelle faisant office de bouchon pour être vissée sur une portée filetée externe du col (1a) du biberon (1).

6. Ensemble d'expression de lait maternel selon l'une quelconque des revendications 1 à 5 , ***caractérisé* en ce que** le clapet anti-retour (3) coopère, après accouplement de l'organe tubulaire de protection (4) sur le biberon, avec une portée circulaire interne (2c) que présente le volume intérieur de l'embase de la téterelle faisant office de bouchon, ladite portée interne étant, d'une manière connue, en communication avec la téterelle (2) par un orifice pour réduire le passage de circulation et de transfert du lait dans le biberon.

7. Ensemble d'expression de lait maternel selon la revendication 2, ***caractérisé* en ce que** l'organe tubulaire de protection (4) incluant le manchon (4b), la portée circulaire d'emmanchement (4a) et la collerette (4c), est réalisé d'une manière monobloc dans une matière plastique alimentaire.

## Patentansprüche

1. Vorrichtung zum Abpumpen von Muttermilch, mit einer Brusthaube (2), die auf einen Gewindeschaft aufgeschraubt wird, die außerhalb eines Halses (1a) gebildet wird, der eine Trinkfläschchenöffnung (1) aufweist, wobei diese Brusthaube (2) über ein Rückschlagventil (3) in Verbindung mit dem Trinkfläschchen (1) steht, ***dadurch gekennzeichnet, dass*** das Rückschlagventil (3) mit einer Schutzvorrichtung (4) fest verbunden ist, die eine runde Dichtfläche (4a) aufweist, die auf die Öffnung des Halses (1a) des Trinkfläschchens aufgepresst werden kann, wobei diese Dichtfläche (4a) koaxial zu einer zylindrischen Hülse (4b) gebildet wird, die das Rückschlagventil (3) umgibt.

2. Vorrichtung zum Abpumpen von Muttermilch gemäß Anspruch 1, ***dadurch gekennzeichnet, dass*** die runde Dichtfläche (4a) einen Kragen (4c) aufweist, der sich auf die Öffnung des Halses des Trinkfläschchens (1) stützt, wenn diese Fläche in diese Öffnung eingepresst wurde.

3. Vorrichtung zum Abpumpen von Muttermilch gemäß einem der vorstehenden Ansprüche 1 bis 2, ***dadurch gekennzeichnet, dass*** die Hülse (4b) und die runde Dichtfläche (4a) eine Innenschulter bilden, die als Sitz zur Befestigung des Rückschlagventils (3) dient.

4. Vorrichtung zum Abpumpen von Muttermilch gemäß einem der vorstehenden Ansprüche 1 bis 3, ***dadurch gekennzeichnet, dass*** der Außenumfang der Dichtfläche (1a) punktuell vertiefte und/oder erhöhte Zonen enthält, um die Verbindung zu erleichtern.

5. Vorrichtung zum Abpumpen von Muttermilch gemäß Anspruch 2, ***dadurch gekennzeichnet, dass*** der Kragen (4c) mit einer runden Dichtung (5) aufliegend zusammenarbeitet, die eine Basisverbindung (2a) der Brusthaube aufweist und die als Stopfen dient, der auf einen externen Gewindeschaft des Halses (1a) des Trinkfläschchens (1) aufgeschraubt werden kann.

6. Vorrichtung zum Abpumpen von Muttermilch gemäß einem der vorstehenden Ansprüche 1 bis 5, ***dadurch gekennzeichnet, dass*** das Rückschlagventil (3) nach Anschluss der rohrförmigen Schutzvorrichtung (4) am Trinkfläschchen, mit einer internen runden Fläche (2c) zusammenarbeitet, die im Innenvolumen der Basisverbindung der Brusthaube vorgesehen ist, die als Stopfen dient, wobei diese Innenfläche in bekannter Art und Weise in Verbindung mit der Brusthaube (2) über eine Öffnung steht, um den Transportweg und die Verbringung der Milch in das Trinkfläschchen zu verkürzen.

7. Vorrichtung zum Abpumpen von Muttermilch gemäß Anspruch 2, ***dadurch gekennzeichnet, dass*** die rohrförmige Schutzvorrichtung (4), die die Hülse (4b), die runde Dichtfläche (4a) und den Hals (4c) umfasst, einteilig aus Lebensmittelkunststoff ausgeführt wird.

## Claims

1. Maternal milk feeding system incorporating a teat (2) screwed onto a threaded span formed on the exterior of a neck (1a) provided on an opening for a baby bottle (1), the said teat (2) being in communication with the baby bottle (1) by means of a check valve (3), **characterized in that** the check valve (3) is integral with a protective device (4) having a circular span (4a) that can be force-fitted into the opening in the neck (1a) of the baby bottle, the said span (4a) being formed coaxially to a cylindrical sleeve (4b) surrounding the check valve (3).

2. Maternal milk feeding system in accordance with claim 1, **characterized in that** the circular span (4a) has a collar (4c) able to bear on the edge of the opening in the neck of the baby bottle (1), after force-fitting of the said span into the said opening.

3. Maternal milk feeding system in accordance with any one of claims 1 or 2, **characterized in that** the sleeve (4b) and the circular force-fitting span (4a) bound an interior shoulder acting as a seat for the securing of the check valve (3).

4. Maternal milk feeding system in accordance with any one of claims 1 to 3, **characterized in that** the exterior periphery of the force-fitting span (1a) has intermittent embossed and/or relief areas to enhance the coupling.

5. Maternal milk feeding system in accordance with claim 2, **characterized in that** the collar (4c) interoperates in contact with a circular seal (5) provided on a base (2a) for the teat acting as a cap designed to be screwed onto an exterior threaded span on the neck (1a) of the baby bottle (1).

6. Maternal milk feeding system in accordance with any one of claims 1 to 5, **characterized in that** the check valve (3) interoperates, after coupling of the tubular protective device (4) onto the baby bottle, with an interior circular span (2c) in the interior volume of the base of the teat acting as a cap, said interior span being, in a known manner, in communication with the teat (2) through an orifice to reduce the flow and transfer of milk into the baby bottle.

7. Maternal milk feeding system in accordance with claim 2, **characterized in that** the tubular protective device (4) incorporating the sleeve (4b), the circular force-fitting span (4a) and the collar (4c) is manufactured as one piece in an alimentary-standard plastic material.
